# EUROPÄISCHE PATENTANMELDUNG

(11) **EP 4 458 454 A1**
(43) Veröffentlichungstag der Anmeldung: **06.11.2024**
(21) Anmeldenummer: 24173173.6
(22) Anmeldetag: 29.04.2024
(51) Int. Cl.: B01D 61/04, B01D 61/10, A61M 1/16, C02F 1/44

(54) **VORRICHTUNG UND VERFAHREN ZUR TEMPERIERUNG VON WEICHWASSER UND/ODER VON PERMEAT FÜR EINE DIALYSEANLAGE**

(30) Priorität: 03.05.2023 DE 102023111496
(71) Anmelder: B. Braun Avitum AG, 34212 Melsungen (DE)
(72) Erfinder: Odernheimer, Philipp, 34212 Melsungen (DE); Lieb, Nino, 34212 Melsungen (DE); Giesa, Jonas, 34121 Kassel (DE)
(74) Vertreter: Tergau & Walkenhorst Intellectual Property GmbH

(57) **Zusammenfassung**

Aufgabe der Erfindung ist es eine Vorrichtung anzugeben, die mit geringen Betriebskosten eine zuverlässige und flexibel an wechselsenden Bedarf anpassbare Temperierung von Weichwasser und/oder von Permeat für Dialyseanwendungen ermöglicht. Dies wird erfindungsgemäß erreicht durch eine Vorrichtung (10) zur Temperierung von Weichwasser in einer Weichwasserzufuhrleitung (2) und/oder von Permeat in einer Permeatentnahmeleitung (6) einer auf dem Prinzip der Umkehrosmose beruhenden und zum Einsatz in einer Dialyseanlage ausgelegten Wasseraufbereitungsanlage (4), umfassend einen wärmeflussmäßig mit einer Wärmequelle (12) und/oder Wärmesenke gekoppelten, ein fluides Wärmeträgermedium aufnehmenden oder enthaltenden Pufferspeicher (16) für Wärme, sowie
• einen primärseitig mittels eines Pumpenkreislaufs (30) für das Wärmeträgermedium an den Pufferspeicher (16) angeschlossenen Weichwasserwärmeübertrager (22), der sekundärseitig in die Weichwasserzufuhrleitung (4) geschaltet ist, und
• einen primärseitig mittels eines Pumpenkreislaufs (32) für das Wärmeträgermedium an den Pufferspeicher (16) angeschlossenen Permeatwärmeübertrager (24), der sekundärseitig in die Permeatentnahmeleitung (6) geschaltet ist.

## Beschreibung

Die Erfindung betrifft eine Vorrichtung zur Temperierung von Weichwasser in einer Weichwasserzufuhrleitung und/oder von Permeat in einer Permeatentnahmeleitung einer auf dem Prinzip der Umkehrosmose beruhenden und zum Einsatz in einer Dialyseanlage ausgelegten Wasseraufbereitungsanlage. Die Erfindung betrifft ferner zugehöriges Betriebsverfahren.

Eine Dialyseanlage zur extrakorporalen Blutwäsche benötigt zum Betrieb hochreines Wasser, auch Permeat genannt, welches üblicherweise in einer Wasseraufbereitungsanlage gemäß dem Prinzip der Umkehrosmose aus enthärtetem Wasser, sogenanntem Weichwasser, gewonnen wird. Enthärtet bedeutet dabei, dass dem Weichwasser Stoffe entzogen wurden, die für die Verkalkung von Rohrleitungen verantwortlich sind. Die Wasseraufbereitungsanlage kann ein integraler Bestandteil oder eine zugeordnete Komponente der Dialyseanlage sein und weist eine Weichwasserzufuhrleitung oder kurz Weichwasserzuleitung und eine Permeatentnahmeleitung oder kurz Permeatableitung auf. Die Permeatentnahmeleitung führt typischerweise zu einer Ringleitung, an die mobile Dialysegeräte als Verbraucher von Permeat anschließbar sind.

Für einen energieeffizienten Betrieb ist es wünschenswert, sowohl das Weichwasser in der Weichwasserzufuhrleitung als auch das Permeat in der Permeatentnahmeleitung zentral zu temperieren bzw. zu klimatisieren (Vorwärmen oder Kühlen), was entsprechend energieaufwändig ist. Speziell bei stark schwankende Permeatabnahme muss die bereitzustellende Heiz- oder Kühlleistung für die Lastspitzen größer sein als bei kontinuierlicher Abnahme notwendig. Insbesondere bei integrierter Heißreinigung kann ein starkes Aufheizen über den Normalbetrieb hinaus erforderlich sein.

Aktuelle Systeme, um die Permeattemperatur in der Ringleitung zu beeinflussen, sind auf das rein elektrische Erhitzen von Permeat zur thermischen Desinfektion beschränkt. Ein zentrales Vorwärmen des Permeats während des Dialysebetriebs ist nicht vorgesehen. Ein zentrales Kühlen des Permeats ist ebenfalls nicht möglich. Die aktuellen Systeme haben keine Interaktion mit dem Weichwasser und können dieses entsprechend auch nicht klimatisieren.

Aufgabe der Erfindung ist es, eine Vorrichtung der genannten Art anzugeben, die mit geringen Betriebskosten eine zuverlässige und flexibel an wechselsenden Bedarf anpassbare Temperierung von Weichwasser und/oder von Permeat für Dialyseanwendungen ermöglicht. Des Weiteren soll ein besonders vorteilhaftes Betriebsverfahren für solch eine Vorrichtung angegeben werden.

Die zuerst genannte Aufgabe wird erfindungsgemäß gelöst durch eine Vorrichtung mit den Merkmalen von Anspruch 1.

Dementsprechend ist eine Vorrichtung zur Temperierung von Weichwasser in einer Weichwasserzufuhrleitung und/oder von Permeat in einer Permeatentnahmeleitung einer auf dem Prinzip der Umkehrosmose beruhenden und zum Einsatz in einer Dialyseanlage ausgelegten Wasseraufbereitungsanlage vorgesehen, umfassend einen wärmeflussmäßig mit einer Wärmequelle und/oder Wärmesenke gekoppelten oder koppelbaren, ein fluides Arbeitsmedium (auch Wärmeträgermedium oder Puffermedium oder Pufferspeichermedium genannt) als Wärmeträger aufnehmenden oder enthaltenden Pufferspeicher für Wärme, sowie
- einen primärseitig mittels eines Pumpenkreislaufs für das Wärmeträgermedium an den Pufferspeicher angeschlossenen Weichwasserwärmeübertrager, der sekundärseitig in die Weichwasserzufuhrleitung geschaltet ist, und
- einen primärseitig mittels eines Pumpenkreislaufs für das Wärmeträgermedium an den Pufferspeicher angeschlossenen Permeatwärmeübertrager, der sekundärseitig in die Permeatentnahmeleitung geschaltet ist.

Durch die Nutzung eines thermischen Pufferspeichers, der sowohl mit der Weichwasserzufuhrleitung als auch mit der Permeatentnahmeleitung der Wasseraufbereitungsanlage thermisch gekoppelt oder koppelbar ist, und der seinerseits wärmeflussmäßig mit einer externen Wärmequelle und/oder Wärmesenke gekoppelt oder koppelbar ist, ist eine Senkung der Betriebskosten durch Nutzung von Umweltenergie oder anderen Systemen zur kostengünstigen Wärmebereitstellung oder Wärmeabfuhr ermöglicht. Das System ist speziell für stark schwankende Permeatabnahme und damit verbundene Schwankungen vom thermischen Energiebedarf ausgelegt. Durch die Kombination eines zur Pufferung ausgelegten thermischen Energiespeichers und durch Anpassungen der Konvektionsströmungen auf den Primärseiten der Wärmeübertrager kann ein schwankender thermische Energiebedarf ausgeregelt werden. Durch Modularität ist eine bedarfsgerechte Anpassung an und Integration in vielfältige Dialyseanlagen, abhängig von den konkreten Anforderungen und von der Einbausituation, erreichbar.

Vorteilhafte Ausgestaltungen sind Gegenstand der abhängigen Ansprüche und der nachfolgenden Beschreibung.

In vorteilhafter Ausgestaltung ist die Wärmequelle und/oder Wärmesenke eine Wärmepumpe, vorzugsweise eine Luft-Wasser-Wärmepumpe. Eine Luft-Wasser-Wärmepumpe nutzt die Umgebungsluft als Wärmequelle. Im Betrieb saugt ein Ventilator die Luft aktiv an und leitet sie an einen Wärmeübertrager, den Verdampfer, weiter. In diesem zirkuliert ein Kältemittel, das aufgrund seiner thermischen Eigenschaften seinen Aggregatzustand bereits bei geringer Temperatur ändert. Tauscht sich das Kältemittel mit der zugeführten Außenluft wärmeflussmäßig aus, erwärmt es sich so lange, bis es schließlich verdampft. Ein nachgeschalteter, elektrisch angetriebener Verdichter erhöht unter Einsatz elektrischer Energie den Druck und die Temperatur des Kältemitteldampfes. Hat der Kältemitteldampf das gewünschte Temperaturniveau erreicht, strömt er weiter zum nächsten Wärmeübertrager, dem Verflüssiger. Hier überträgt er seine Wärme auf ein flüssiges Arbeitsmedium, in der Regel Wasser, und kondensiert. Das so erwärmte Wasser wird nun im Rahmen der vorliegenden Erfindung, typischerweise durch eine Pumpe, in den Speicherbehälter des Pufferspeichers geleitet und steht dort als fluides Arbeitsmedium bzw. Wärmeträger zur Verfügung, um die gespeicherte Wärmeenergie bedarfsgerecht auf das Weichwasser und/oder das Permeat zu übertragen.

Das im Rahmen der Erfindung verwendete Arbeitsmedium ist vorzugsweise Wasser, ggf. mit Zusätzen, oder eine wässrige Lösung, aber auch andere Medien mit geeigneten thermischen Eigenschaften sind möglich.

Das Arbeitsmedium innerhalb des Pufferspeichers (auch: Pufferspeichermedium) ist bevorzugt so gestaltet bzw. beschaffen, dass eine Detektion des Arbeitsmediums im Permeat über elektrische oder optische Messungen möglich ist, um auf diese Weise eine Leckage (insbesondere innerhalb des Permeatwärmeübertragers) in die Permeatleitung(en) und eine damit einhergehende Verunreinigung des Permeats zu erkennen. Vorteilhafterweise ist die erfindungsgemäße Vorrichtung daher mit geeigneten Detektoren ausgestattet, die über die Anlagensteuerung eine entsprechende Warnung ausgeben und ggf. die Permeatabgabe unterbrechen können.

Mit anderen Worten ist das Arbeits- bzw. Puffermedium vorteilhafterweise so gestaltet, dass ein in ihm enthaltener Stoff (idealerweise biologisch unschädlich) durch einen Sensor detektiert werden kann. Beispiel: Das Puffermedium ist stark mit Na+ Ionen versetzt. Sobald das Na+-haltige Puffermedium in das Permeat gelangt, kann dieses ionenspezifisch/ionenunspezifisch über ein Amperemeter bzw. eine Leitfähigkeitsmesselektrode detektiert werden. Alternativ oder zusätzlich könnte ein Farbstoff und/oder ein optisch aktiver Stoff in das Puffermedium gegeben werden, damit die Substanz bei Eintreten in das Permeat optisch detektiert werden kann.

Vorzugsweise erfolgt der Transport des Arbeitsmediums von der Wärmepumpe zum Pufferspeicher und zurück in einem Kreislauf, angetrieben durch eine oder mehrere Pumpen, wobei zusätzlich oder alternativ ein passiver Konvektionstransport möglich ist.

Vorteilhafterweise ist die Wärmepumpe in ihrer Kreislaufrichtung umkehrbar, kann also auch als Kältemaschine bzw. Wärmesenke eingesetzt werden, um bei Bedarf das Arbeitsmedium - und damit dann das Weichwasser und/oder das Permeat - zu kühlen.

In einer vorteilhaften Variante sind mehrere Wärmepumpen parallel als Wärmequelle und/oder Wärmesenke an den Pufferspeicher angeschlossen, um auf diese Weise die maximale Wärme- oder Kälteleistung zu erhöhen, und um gemäß den Prinzipien von Redundanz und/oder Diversität verschiedenartige Wärme- und/oder Kälte-Reservoirs zu erschließen.

Im Sinne einer besonders kompakten Lösung kann der Weichwasserwärmeübertrager und/oder der Permeatwärmeübertrager baulich in den Pufferspeicher integriert sein.

Die im System verwendeten Wärmeübertrager, das heißt insbesondere der Weichwasserwärmeübertrager und/oder der Permeatwärmeübertrager, können für eine effiziente Wärmeübertragung vorteilhafterweise als Rohrwendel-Wärmeübertrager ausgestaltet sein. Derartige Rohrwendelwärmeübertrager können insbesondere als Wellrohrwärmeübertrager ausgestaltet sein.

Vorteilhafterweise ist eine den Weichwasserwärmeübertrager umgehende Bypassleitung in die Weichwasserzufuhrleitung geschaltet. Entsprechend ist vorteilhafterweise eine den Permeatwärmeübertrager umgehende Bypassleitung in die Permeatentnahmeleitung geschaltet. Durch geeignete Einstellung des Mischungsverhältnisses aus Wärmeübertragerstrom und Umgehungsstrom mittels Regelventilen oder dergleichen lässt sich die Temperatur des Weichwassers oder Permeats einfach, schnell und in einem gewissen Maße unabhängig von der Temperatur des Arbeitsmediums im Pufferspeicher einstellen. Alternativ oder zusätzlichen zu den Ventilen können auch Pumpen verwendet werden, um die Mischung von temperiertem (den jeweiligen Wärmeübertrager durchlaufendem) und nicht-temperiertem (den jeweiligen Bypass durchlaufendem) Permeat / Weichwasser zu steuern oder zu regeln.

Vorteilhafterweise weist der jeweilige Pumpenkreislauf eine drehzahlvariable Pumpe für das Arbeitsmedium auf, um die primärseitig im Weichwasserwärmeübertrager und/oder im Permeatwärmeübertrager zur Verfügung gestellte Wärmezufuhr oder -abfuhr bedarfsgerecht einstellen oder regeln zu können.

In einer bevorzugten Variante ist in die Permeatentnahmeleitung eine Zusatzheizung zur Erwärmung des Permeats auf eine zum thermischen Desinfizieren nachfolgender Leitungsabschnitte ausreichende Desinfektionstemperatur geschaltet. Diese Zusatzheizung ist bevorzugt eine elektrische Heizung. Die Zusatzheizung ist in Bezug auf das Permeat bevorzugt stromabwärts des Permeatwärmeübertragers angeordnet. Durch die Vorwärmung des Permeats mit Hilfe des Pufferspeichers muss die Zusatzheizung nur die verbleibende Temperaturdifferenz zwischen der Temperatur des Permeats am Ausgang des Permeatwärmeübertragers und der gewünschten Desinfektionstemperatur in der Ringleitung heiztechnisch bewerkstelligen.

Erfindungsgemäß wird in dem jeweiligen Pumpenkreislauf das Wärmeträgermedium aus dem Pufferspeicher (und nicht etwa ein separates Arbeitsmedium) zirkuliert. Das heißt, das Wärmeträgermedium wird dem Pufferspeicher entnommen, über den jeweiligen Pumpenkreislauf zum Weichwasserwärmeübertrager / Permeatwärmeübertrager geführt und von dort wieder in den Pufferspeicher zurückgeführt. Insofern handelt es sich jeweils um einen Pumpenkreislauf für Wärmeträgermedium aus dem Pufferspeicher. Der Weichwasserwärmeübertrager / Permeatwärmeübertrager wird also primärseitig von dem Wärmeträgermedium aus dem Pufferspeicher durchströmt.

Die Erfindung betrifft weiterhin eine Dialyseanlage mit einer auf dem Prinzip der Umkehrosmose beruhenden Wasseraufbereitungsanlage, die eine Weichwasserzufuhrleitung und eine Permeatentnahmeleitung aufweist, und die mit einer Vorrichtung der oben beschriebenen Art ausgestattet oder verbindbar ist, nämlich zur Temperierung von Weichwasser in der Weichwasserzufuhrleitung und/oder von Permeat in der Permeatentnahmeleitung.

Beim Betrieb der oben beschriebenen Vorrichtung ist es von Vorteil, wenn der Wärmefluss von der Wärmequelle zum Pufferspeicher oder vom Pufferspeicher zur Wärmesenke in Abhängigkeit von einer im Pufferspeicher gemessenen Temperatur des Arbeitsmediums geregelt wird. Genauer gesagt wird die Entscheidung, ob die Wärmepumpe als Senke oder Quelle dient, vorzugsweise über einen Temperatursensor am Weichwassereingang (vor dem Durchströmen des Weichwasserwärmeübertragers) getroffen, hängt also von der Eingangstemperatur des Weichwassers ab. Die anschließende Regelung der Wärmepumpe erfolgt dann wie erwähnt zweckmäßigerweise über die Temperatur des Arbeitsmediums im Pufferspeicher.

In einer als eigenständig erfinderisch angesehenen und von der im letzten Absatz beschriebenen Verfahrensführung unabhängigen Ausgestaltung weist der Pumpenkreislauf mit dem Weichwasserwärmeübertrager primärseitig eine drehzahlvariable Pumpe für das Arbeitsmedium auf, deren Drehzahl in Abhängigkeit von einer sekundärseitig stromabwärts des Weichwasserwärmeübertragers gemessenen Weichwassertemperatur geregelt wird.

In einer ebenfalls als eigenständig erfinderisch und unabhängig angesehenen Ausgestaltung weist der Pumpenkreislauf mit dem Permeatwärmeübertrager primärseitig eine drehzahlvariable Pumpe für das Arbeitsmedium auf, deren Drehzahl in Abhängigkeit von einer sekundärseitig stromabwärts des Permeatwärmeübertragers gemessenen Permeattemperatur geregelt wird.

In einer Variante, die insbesondere im Fall der oben erwähnten Zusatzheizung von Vorteil ist, wird das Permeat während eines Desinfektionsvorgangs auf eine Desinfektionstemperatur erwärmt und nach erfolgter Desinfektion eines Leitungsabschnitts durch den Permeatwärmeübertrager (zurück) geleitet, wobei das Permeat dabei vorhandene Restwärme an den Pufferspeicher abgibt. Die zurückgewonnene Wärme steht dann erneut für verschiedenartige Anwendungen zur Verfügung.

In einer vorteilhaften Ausgestaltung dieser Kühlung nach erfolgter Heißdesinfektion ist als Wärmesenke eine Wärmepumpe mit einem Verdichter, einem Wärmeübertrager und einem dem Wärmeübertrager zugeordneten Ventilator vorgesehen, wobei das (Rück-) Kühlen des Pufferspeichers nach einer Desinfektion aktiv oder passiv durch die Wärmepumpe erfolgt bzw. unterstützt wird. Bei der aktiven Variante arbeitet die Wärmepumpe bei laufendem Verdichter an der Kühlung mit; bei der passiven Variante wird nur der Wärmeübertrager der Wärmepumpe (Verdichter ausgeschaltet / Ventilator eingeschaltet) zur Wärmeabgabe an die Umgebung genutzt.

Zusammengefasst beinhaltet die Erfindung eine Vorrichtung und ein Verfahren zur Kopplung einer Permeat- und/oder Weichwasserleitung einer Wasseraufbereitungsanlage über jeweils einen Wärmeübertrager mit einem Pufferspeicher eines zentralen oder dezentralen Wärmeerzeugers zur Heizung oder Kühlung der Fluide innerhalb der Leitungen. Es ist damit ein modulares System verwirklicht zum zentralen
- Temperieren bzw. Klimatisieren (Vorwärmen/ Kühlen) von Permeat während der Dialysebehandlung,
- thermischen Desinfizieren von mindestens einer Permeat-Ringleitung und angeschlossener Dialysemaschine & Umkehrosmoseanlage, und/oder
- Temperieren bzw. Klimatisieren (Vorwärmen/ Kühlen) von Weichwasser vor Eintritt in die Umkehrosmoseanlage.

Mehrere Ausführungsbeispiele der Erfindung werden nachfolgend unter Bezugnahme auf die beigefügten Zeichnungen erläutert. Es zeigt:
- FIG. 1: schematisch eine Vorrichtung zur Temperierung von Weichwasser in einer Weichwasserzufuhrleitung und/oder von Permeat in einer Permeatentnahmeleitung einer auf dem Prinzip der Umkehrosmose beruhenden und zum Einsatz in einer Dialyseanlage ausgelegten Wasseraufbereitungsanlage,
- FIG. 2: eine Abwandlung der Vorrichtung aus FIG. 1, und
- FIG. 3: eine weitere Abwandlung der Vorrichtung aus FIG. 1.

Gleiche oder gleich wirkende Elemente sind in allen Figuren mit denselben Bezugszeichen versehen.

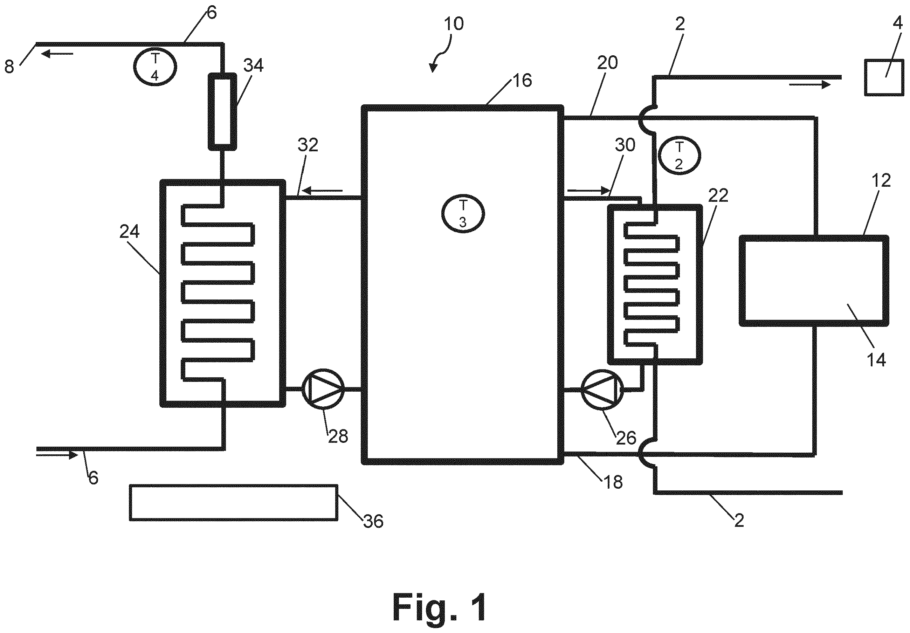

In FIG. 1 ist das Vorrichtungs- und Verfahrensschema der bevorzugten Ausgestaltung schematisch dargestellt. Enthärtetes Wasser, Weichwasser genannt, wird über eine Weichwasserzufuhrleitung 2 einer nur schematisch angedeuteten Wasseraufbereitungsanlage 4 zugeführt und dort gemäß dem Prinzip der Umkehrosmose für Dialyseanwendungen aufbereitet und gereinigt. Das hochreine Wasser, Permeat genannt, verlässt die Wasseraufbereitungsanlage 4 über eine Permeatentnahmeleitung 6 und wird im Beispiel über diese einer Permeat-Ringleitung 8 zugeführt, an die mobile Dialysegeräte als Verbraucher von Permeat anschließbar sind.

Das beispielsweise mit einer Temperatur von 15 °C aus der vorgeschalteten Enthärtungsstufe (nicht dargestellt) kommende Weichwasser muss für eine effiziente Durchführung der Umkehrosmose auf eine Temperatur von beispielsweise 25 °C erwärmt werden. Analog muss das die Wasseraufbereitungsanlage 4 mit einer Temperatur von beispielsweise 25 °C (entsprechend der dortigen Weichwassertemperatur) verlassende Permeat vor der Verwendung als Dialyseflüssigkeit auf eine Temperatur von beispielsweise 36 °C (Körpertemperatur) erwärmt werden. In anderen Szenarien kann eine Kühlung der Flüssigkeiten erforderlich sein. Zu diesem Zweck ist eine Vorrichtung 10 zur Temperierung von Weichwasser und/oder von Permeat vorgesehen, die nachfolgend näher beschrieben wird.

Von einem als Wärmequelle 12 wirksamen Wärmeerzeuger, in der bevorzugten Ausführung eine Wärmepumpe 14, insbesondere eine Luft-Wasser-Wärmepumpe, wird die der Umwelt entnommene thermische Energie (Q_{zu}) in Form von Wärme dem Wärme- oder Pufferspeicher 16 zugeführt. Der Pufferspeicher 16 umfasst einen thermisch isolierten Behälter für ein fluides Speicher- bzw. Arbeitsmedium, welches als Wärmeträger wirksam ist, im Beispiel im wesentlichen Wasser. Der Pufferspeicher 16 und die Wärmepumpe 14 sind hier im Beispiel Bestandteile eines geschlossenen Kreislaufs für das Arbeitsmedium: Vergleichsweise kaltes Arbeitsmedium wird über die Kaltmediumleitung 18 dem Pufferspeicher 16 entnommen, durch eine die Wärmepumpe 14 enthaltende Heizstrecke geführt, und schließlich über die Heißmediumleitung 20 wieder in den Pufferspeicher 16 eingespeist. Zum Transport des Arbeitsmediums kann eine (nicht dargestellte) Umwälzpumpe in den Kreislauf geschaltet sein, ggf. verwirklicht durch die Wärmepumpe 16 selbst. Im Ausführungsbeispiel beträgt die Soll- oder Zieltemperatur des Arbeitsmediums im Pufferspeicher 16 etwa 40 °C.

An den Pufferspeicher 16 sind primärseitig zwei Wärmeübertrager 22, 24 über Rohrleitungen angeschlossen. In den Rohrleitungen sind drehzahlvariable Pumpen 26, 28 angeordnet. Damit sind primärseitig zwei Pumpenkreisläufe 30, 32 für das Arbeitsmedium, nämlich aus dem Pufferspeicher 16 über den jeweiligen Wärmeübertrager 22, 24 und wieder in den Pufferspeicher 16 zurück, verwirklicht. Der sekundärseitig in die Weichwasserzufuhrleitung 2 geschaltete Weichwasserwärmeübertrager 22 wird wie bereits erwähnt zur Erwärmung des Weichwassers, welches nachfolgend in die innerhalb der Wasseraufbereitungsanlage 4 verwirklichte Umkehrosmoseanlage geleitet wird, verwendet. Durch eine Anhebung der Weichwassereingangstemperatur sinkt beim Betrieb der Umkehrosmoseanlage der spezifische Energiebedarf, gekennzeichnet durch den Energieverbrauch der Osmosepumpe. Der sekundärseitig in die Permeatentnahmeleitung 6 geschaltete Permeatwärmeübertrager 24 hingegen wird verwendet, um während der Dialysebehandlung das Permeat vor Eintritt in die Ringleitung 8 bzw. den Dialysemaschinen auf eine Solltemperatur zu erwärmen.

Bei einer thermischen Desinfektion der Ringleitung 8 durch heißes Permeat kann die Speichertemperatur, d. h. die Temperatur des Arbeitsmediums im Pufferspeicher 16, durch den Wärmeerzeuger bzw. die Wärmepumpe 14 auf ein technisch sinnvolles Maß erhöht werden. Eine Nachheizung auf die geforderte Solltemperatur wird durch eine Zusatzheizung 34, in der bevorzugten Ausführung eine elektrische Zusatzheizung, erreicht. Die Zusatzheizung 34 ist zu diesem Zweck stromabwärts des Permeatwärmeübertragers 24 in die Permeatentnahmeleitung 6 geschaltet. Nach erfolgter Desinfektion kann ein Teil der zuvor zugeführten Energie vom Permeat durch den Permeatwärmeübertrager 24 auf das Speicher- bzw. Arbeitsmedium des Energie- bzw. Pufferspeichers 16 übertragen werden. Die zurückgewonnene Energie kann nachfolgend zum Vorwärmen vom Weichwasser und/oder dem Permeat verwendet werden.

In sehr warmen Regionen, in denen die Weichwassertemperatur eingangsseitig über einem definierten Grenzwert liegt, kann durch Kreislaufumkehr der Wärmepumpe 14 dem Wärme- bzw. Pufferspeicher 16 Energie entzogen und an die Umgebung abgegeben werden (Q_{ab}), wodurch das Weichwasser und/oder das Permeat beim Durchströmen durch die Wärmeübertrager 22, 24 gekühlt werden. Dadurch kann der Wasserbedarf in Folge von Temperaturverwurf der Umkehrosmoseanlage verringert werden.

Die Steuerung und Regelung der einzelnen Aktoren kann von einer hier nur schematisch angedeuteten zentralen Steuerungseinheit 36 erfolgen oder dezentral realisiert werden. Eine bevorzugte Regelung der einzelnen Aktoren ist unter Berücksichtigung der jeweiligen Prozessgrößen im Nachfolgenden aufgelistet:
- Der Wärmeerzeuger bzw. die Wärmepumpe 14 wird durch Aufnahme und Auswertung von Messdaten des Temperatursensor T3, welcher die Temperatur des Speichermediums im Pufferspeicher 16 misst, geregelt.
- Die Pumpe 26 im Pumpenkreislauf 30 für die Weichwassertemperierung wird durch Aufnahme und Auswertung von Messdaten des Temperatursensors T2 geregelt. Der Temperatursensor T2 ist stromabwärts des Weichwasserwärmeübertragers 22, z. B. an dessen Ausgang, in die Weichwasserzufuhrleitung 2 geschaltet.
- Die Pumpe 28 im Pumpenkreislauf 32 für die Permeattemperierung wird durch Aufnahme und Auswertung von Messdaten des Temperatursensors T4 geregelt. Der Temperatursensor T4 ist stromabwärts des Permeatwärmeübertragers 24, vorzugsweise stromabwärts der Zusatzheizstrecke mit der Zusatzheizung 34, in die Permeatentnahmeleitung 6 geschaltet.
- Die Zusatzheizstrecke, sprich die Zusatzheizung 34, wird durch Aufnahme und Auswertung von Messdaten des am Ausgang der Zusatzheizstrecke oder weiter stromabwärts angeordneten Temperatursensors T4 geregelt.

Die in FIG. 1 dargestellte Vorrichtung 10 kann eine Reihe von Abwandlungen erfahren, die im Rahmen der beanspruchten Erfindung liegen, und die - soweit technisch sinnvoll - beliebig miteinander kombiniert werden können. Insbesondere können die folgenden Abwandlungen realisiert werden:
- Die benötigte Wärme kann auch durch eine andere Art von dezentralem oder zentralem Wärmeerzeuger anstelle der Wärmepumpe 14 bereitgestellt werden.
- Die benötigte Wärme, die von der elektrischen Zusatzheizung 34 bereitgestellt wird, kann auch durch eine andere Art von dezentralem oder zentralem Wärmeerzeuger bereitgestellt werden.
- Durch den modularen Aufbau kann das System auch ohne Weichwasserwärmeübertrager 22 oder Permeatwärmeübertrager 24 ausgeführt werden.
- Wenn keine thermische Desinfektion gefordert ist, kann die Zusatzheizung 34 entfallen.
- Wenn der Wärmeerzeuger bzw. die Wärmepumpe 14 technisch sinnvoll die erforderliche Desinfektionstemperatur bereitstellen kann, ist eine Zusatzheizung 34 nicht erforderlich.

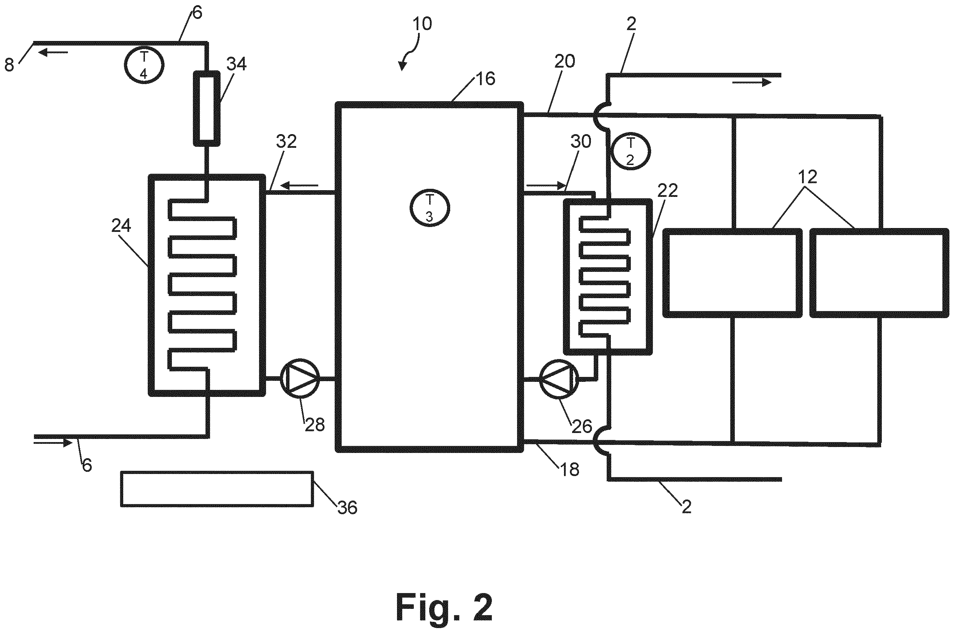

Bei höherem Wärmebedarf können gemäß FIG. 2 mehrere Wärmeerzeuger, insbesondere Wärmepumpen 14, parallel an den Wärme- bzw. Pufferspeicher 16 angeschlossen werden. Diese Variante kann mit allen anderen kombiniert werden.

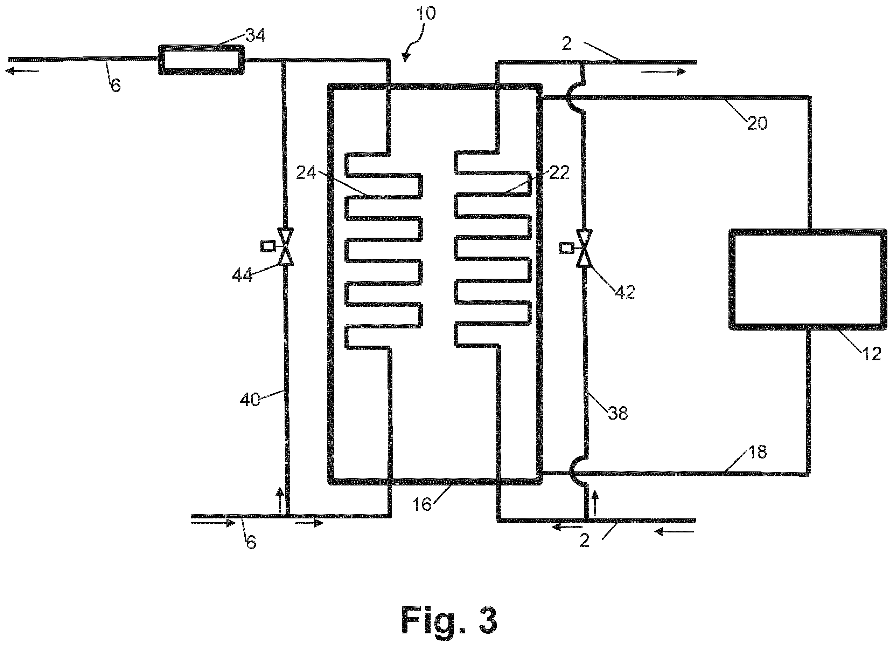

In einer anderen Ausführungsform gemäß FIG. 3 sind die Wärmeübertrager 22, 24 in den Pufferspeicher 16 integriert. Die primärseitigen Pumpenkreisläufe 30, 32 mit den Pumpen 26, 28 können hier entfallen, weil die Wärmeübertrager (z. B. Wellrohre) bereits im Speichermedium (Wärmeträgermedium) integriert sind. Auch die Heizstrecke mit der Zusatzheizung 34 kann optional im System integriert werden. Auch diese Varianten können mit allen anderen kombiniert werden.

Damit es bei geringer Wasserabnahme (Permeat und/oder Weichwasser) und einer erhöhten Speichertemperatur nicht zu einer unzulässigen Temperaturerhöhung kommt, kann wie in FIG. 3 dargestellt über die Bypässe bzw. Bypassleitungen 38, 40 kälteres Wasser eingemischt werden, um die geforderte Solltemperatur einzuhalten. Die dazu erforderlichen Ventile 42, 44 im Leitungssystem können beispielhaft als Mehr-Wege-Ventile (an den Knotenpunkten oder Leitungsverzweigungen oder Leitungsvereinigungen), Regelventile oder Magnetventile ausgeführt sein. Auch diese Varianten können mit allen anderen der weiter oben beschriebenen Varianten kombiniert werden.

### Bezugszeichenliste

- 2: Weichwasserzufuhrleitung
- 4: Wasseraufbereitungsanlage
- 6: Permeatentnahmeleitung
- 8: Ringleitung
- 10: Vorrichtung
- 12: Wärmequelle
- 14: Wärmepumpe
- 16: Pufferspeicher
- 18: Kaltmediumleitung
- 20: Heißmediumleitung
- 22: Weichwasserwärmeübertrager
- 24: Permeatwärmeübertrager
- 26: Pumpe
- 28: Pumpe
- 30: Pumpenkreislauf
- 32: Pumpenkreislauf
- 34: Zusatzheizung
- 36: Steuerungseinheit
- 38: Bypassleitung
- 40: Bypassleitung
- 42: Ventil
- 44: Ventil

- T2...T4: Temperatursensoren / Temperaturen

## Patentansprüche

1. Vorrichtung (10) zur Temperierung von Weichwasser in einer Weichwasserzufuhrleitung (2) und/oder von Permeat in einer Permeatentnahmeleitung (6) einer auf dem Prinzip der Umkehrosmose beruhenden und zum Einsatz in einer Dialyseanlage ausgelegten Wasseraufbereitungsanlage (4), umfassend einen wärmeflussmäßig mit einer Wärmequelle (12) und/oder Wärmesenke gekoppelten, ein fluides Wärmeträgermedium aufnehmenden oder enthaltenden Pufferspeicher (16) für Wärme, sowie
• einen primärseitig mittels eines Pumpenkreislaufs (30) für das Wärmeträgermedium an den Pufferspeicher (16) angeschlossenen Weichwasserwärmeübertrager (22), der sekundärseitig in die Weichwasserzufuhrleitung (4) geschaltet ist, und
• einen primärseitig mittels eines Pumpenkreislaufs (32) für das Wärmeträgermedium an den Pufferspeicher (16) angeschlossenen Permeatwärmeübertrager (24), der sekundärseitig in die Permeatentnahmeleitung (6) geschaltet ist.

2. Vorrichtung (10) nach Anspruch 1, wobei die Wärmequelle (12) und/oder Wärmesenke eine Wärmepumpe (14), vorzugsweise eine Luft-Wasser-Wärmepumpe, ist.

3. Vorrichtung (10) nach Anspruch 2, wobei die Wärmepumpe (14) in ihrer Kreislaufrichtung umkehrbar ist.

4. Vorrichtung (10) nach Anspruch 3 mit einer Steuer- oder Regeleinrichtung, die die Kreislaufrichtung der Wärmepumpe (14) anhand einer in der Weichwasserzufuhrleitung (4) stromaufwärts des Weichwasserwärmeübertragers (22) gemessenen Weichwasserzulauftemperatur einstellt.

5. Vorrichtung (10) nach einem der Ansprüche 2 bis 4, wobei mehrere Wärmepumpen (14) parallel als Wärmequelle (12) und/oder Wärmesenke an der Pufferspeicher (16) angeschlossen sind.

6. Vorrichtung (10) nach einem der vorangehenden Ansprüche, wobei der Weichwasserwärmeübertrager (22) und/oder der Permeatwärmeübertrager (24) baulich in den Pufferspeicher (16) integriert ist.

7. Vorrichtung (10) nach einem der vorangehenden Ansprüche, wobei eine den Weichwasserwärmeübertrager (22) umgehende Bypassleitung (38) in die Weichwasserzufuhrleitung (2) geschaltet ist, und/oder wobei eine den Permeatwärmeübertrager (24) umgehende Bypassleitung (40) in die Permeatentnahmeleitung (6) geschaltet ist.

8. Vorrichtung (10) nach einem der vorangehenden Ansprüche, wobei der jeweilige Pumpenkreislauf (30, 32) eine drehzahlvariable Pumpe (26, 28) für das Wärmeträgermedium aufweist.

9. Vorrichtung (10) nach einem der vorangehenden Ansprüche, wobei in die Permeatentnahmeleitung (6) eine Zusatzheizung (34) zur Erwärmung des Permeats auf eine zum thermischen Desinfizieren nachfolgender Leitungsabschnitte ausreichende Desinfektionstemperatur geschaltet ist.

10. Vorrichtung (10) nach Anspruch 9, wobei die Zusatzheizung (34) eine elektrische Heizung ist.

11. Vorrichtung (10) nach Anspruch 9 oder 10, wobei die Zusatzheizung (34) in Bezug auf das Permeat stromabwärts des Permeatwärmeübertragers (24) angeordnet ist.

12. Dialyseanlage mit einer auf dem Prinzip der Umkehrosmose beruhenden Wasseraufbereitungsanlage (4), die eine Weichwasserzufuhrleitung (2) und eine Permeatentnahmeleitung (6) aufweist, und mit einer Vorrichtung (10) nach einem der vorangehenden Ansprüche zur Temperierung von Weichwasser in der Weichwasserzufuhrleitung (2) und/oder von Permeat in der Permeatentnahmeleitung (6).

13. Verfahren zum Betreiben einer Vorrichtung (10) nach einem der Ansprüche 1 bis 11, wobei der Wärmefluss von der Wärmequelle (12) zum Pufferspeicher (16) oder vom Pufferspeicher (16) zur Wärmesenke in Abhängigkeit von einer im Pufferspeicher (16) gemessenen Temperatur (T3) des Wärmeträgermediums geregelt wird.

14. Verfahren nach Anspruch 13, wobei der Pumpenkreislauf (30) mit dem Weichwasserwärmeübertrager (22) primärseitig eine Pumpe (26) für das Wärmeträgermedium aufweist, deren Drehzahl in Abhängigkeit von einer sekundärseitig stromabwärts des Weichwasserwärmeübertragers (22) gemessenen Weichwassertemperatur (T2) geregelt wird.

15. Verfahren nach Anspruch 13 oder 14, wobei der Pumpenkreislauf (32) mit dem Permeatwärmeübertrager (24) primärseitig eine Pumpe (28) für das Wärmeträgermedium aufweist, deren Drehzahl in Abhängigkeit von einer sekundärseitig stromabwärts des Permeatwärmeübertragers (24) gemessenen Permeattemperatur (T4) geregelt wird.

16. Verfahren nach einem der Ansprüche 13 bis 15, wobei das Permeat auf eine Desinfektionstemperatur erwärmt wird und nach erfolgter Desinfektion eines Leitungsabschnitts durch den Permeatwärmeübertrager (24) geleitet wird und dabei vorhandene Restwärme an den Pufferspeicher (16) abgibt.

17. Verfahren nach Anspruch 16, wobei als Wärmesenke eine Wärmepumpe (14) mit einem Verdichter und einem Ventilator vorgesehen ist, und wobei während oder nach der Kühlung des Permeats die Wärmepumpe (14) als Wärmesenke betrieben wird, insbesondere aktiv mit laufendem Verdichter oder passiv mit ausgeschaltem Verdichter, jedoch mit eingeschaltetem Ventilator.
